# EUROPEAN PATENT APPLICATION

(11) **EP 4 527 390 A1**
(43) Date of publication of application: **26.03.2025**
(21) Application number: 23806969.4
(22) Date of filing: 17.05.2023
(51) Int. Cl.: A61K 31/7068, A61P 31/14

(54) **USE OF EIDD-1931 OR DERIVATIVE THEREOF IN TREATMENT OF ENTEROVIRUS INFECTION**

(30) Priority: 20.05.2022 CN 202210549687
(71) Applicant: Academy of Military Medical Sciences, Beijing 100850 (CN)
(72) Inventor: ZHONG, Wu, Beijing 100850 (CN); CAO, Ruiyuan, Beijing 100850 (CN); LI, Wei, Beijing 100850 (CN); LI, Yuexiang, Beijing 100850 (CN); DAI, Qingsong, Beijing 100850 (CN); LI, Song, Beijing 100850 (CN)
(74) Representative: Lavoix
(86) International application number: PCT/CN2023/094712
(87) International publication number: WO 2023/222018

(57) **Abstract**

The present invention relates to use of a compound represented by formula I, a geometric isomer thereof or a pharmaceutically acceptable salt, a solvate and/or a hydrate thereof and a pharmaceutical composition comprising the compound in the preparation of a drugs for treating diseases or infections caused by enteroviruses.

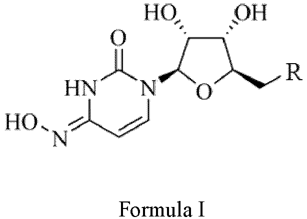

## Description

The present application is based on and claims the benefit of priority from Chinese application No. 202210549687.9, filed on May 20, 2022, the disclosures of which are incorporated herein by reference in its entirety.

### Technical Field

The present invention relates to a use of the compound represented by Formula I, a geometric isomer thereof or a pharmaceutically acceptable salt, a solvate and/or a hydrate thereof, and a pharmaceutical composition comprising the above compound in the treatment of an enterovirus infection.

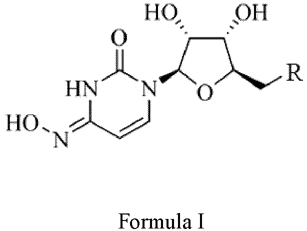

### Background Art

EIDD-1931 (the compound represented by Formula I, wherein R represents hydroxyl) is the active form of the marketed drug Molnupiravir (EIDD-2801, the compound represented by Formula I, wherein R represents isobutyryloxy group). EIDD-1931 has good inhibitory effects against Chikungunya virus (CHIKV), Venezuelan equine encephalitis virus (VEEV), respiratory syncytial virus (RSV), hepatitis C virus (HCV), norovirus (NV), influenza A virus (IAV), influenza B virus (IBV), Ebola virus (EBOV), Middle East respiratory syndrome coronavirus (MERS-CoV) and novel coronavirus (SARS-CoV-2). Molnupiravir (EIDD-2801) is an oral formulation of EIDD-2801. The results of in vivo experiments in mice showed that the preventive use of EIDD-2801 can prevent lung damage caused by MERS-CoV or SARS-CoV infections, and the therapeutic use of EIDD-2801 within 12 hours or 24 hours after infection can significantly reduce the degree of lung damage and weight loss in mice. At present, EIDD-2801 has been approved for marketing in many countries such as the United States and the United Kingdom for the treatment of COVID-19 pneumonia.

Enteroviruses, belonging to the genus *Enterovirus* of the family *Picornaviridae,* are a type of single-stranded positive-sense RNA viruses, and mainly include poliovirus, echovirus, coxsackievirus and novel enterovirus (including EV-A71, EV-D68, etc.). Typical symptoms of enterovirus infection include hand-foot-and-mouth disease, herpangina, aseptic encephalitis, meningitis, poliomyelitis, etc. The diseases and symptoms caused by enterovirus infection are diverse, including mild fever that may resolve itself, and a few that develop into severe illness or even death. At present, the enterovirus infection is mainly treated with mainly symptomatic therapy and supportive therapy in clinic, and no specific antiviral drugs are available.

### Contents of the Invention

The purpose of the present invention is to find a drug with antiviral activity against enterovirus, which can be used for the treatment of a related diseases caused by enterovirus infection, such as hand-foot-and-mouth disease, viral angina, aseptic meningitis, brain stem encephalitis, myocarditis, neurogenic pulmonary edema and central nervous system infection. Through creative research, it is found in the present invention that the compound represented by Formula I, a geometric isomer thereof or a pharmaceutically acceptable salt, a solvate and/or a hydrate thereof has the function of inhibiting the replication of enterovirus and can be used to the treatment of a disease caused by enterovirus.

The present invention relates to a use of the compound represented by Formula I, a geometric isomer thereof or a pharmaceutically acceptable salt, a solvate and/or a hydrate thereof in the manufacture of a medicament for treating a disease or an infection caused by an enterovirus, 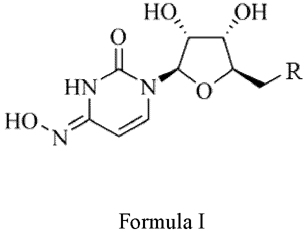 wherein R represents hydroxyl, hydrogen atom, ester group, alkoxy, phenyl, heterocyclyl or alkyl.

The present invention also relates to a use of the compound represented by Formula I, a geometric isomer thereof or a pharmaceutically acceptable salt, a solvate and/or a hydrate thereof in the manufacture of a medicament as an enterovirus inhibitor.

The present invention also relates to a use of the compound represented by Formula I, a geometric isomer thereof or a pharmaceutically acceptable salt, a solvate and/or a hydrate thereof in the manufacture of a medicament for inhibiting the replication or reproduction of enterovirus in a cell.

The present invention also relates to a use of a pharmaceutical composition in the manufacture of a medicament for treating a disease or an infection caused by an enterovirus, wherein the pharmaceutical composition comprises the compound represented by Formula I, a geometric isomer thereof or a pharmaceutically acceptable salt, a solvate and/or a hydrate thereof.

The present invention also relates to a use of a pharmaceutical composition in the manufacture of a medicament as an enterovirus inhibitor, wherein the pharmaceutical composition comprises the compound represented by Formula I, a geometric isomer thereof or a pharmaceutically acceptable salt, a solvate and/or a hydrate thereof.

The present invention also relates to a use of a pharmaceutical composition in the manufacture of a medicament for inhibiting the replication or reproduction of enterovirus in a cell, wherein the pharmaceutical composition comprises the compound represented by Formula I, a geometric isomer thereof or a pharmaceutically acceptable salt, a solvate and/or a hydrate thereof.

The present invention also relates to the compound represented by Formula I, a geometric isomer thereof or a pharmaceutically acceptable salt, a solvate and/or a hydrate thereof, for use in treating a disease or an infection caused by an enterovirus.

The present invention also relates to the compound represented by Formula I, a geometric isomer thereof or a pharmaceutically acceptable salt, a solvate and/or a hydrate thereof, for use as an enterovirus inhibitor.

The present invention also relates to the compound represented by Formula I, a geometric isomer thereof or a pharmaceutically acceptable salt, a solvate and/or a hydrate thereof, for use in inhibiting the replication or reproduction of enterovirus in a cell.

The present invention also relates to a pharmaceutical composition for use in treating a disease or an infection caused by an enterovirus, wherein the pharmaceutical composition comprises the compound represented by Formula I, a geometric isomer thereof or a pharmaceutically acceptable salt, a solvate and/or a hydrate thereof.

The present invention also relates to a pharmaceutical composition for use as an enterovirus inhibitor, wherein the pharmaceutical composition comprises the compound represented by Formula I, a geometric isomer thereof or a pharmaceutically acceptable salt, a solvate and/or a hydrate thereof.

The present invention also relates to a pharmaceutical composition for use in inhibiting the replication or reproduction of enterovirus in a cell, wherein the pharmaceutical composition comprises the compound represented by Formula I, a geometric isomer thereof or a pharmaceutically acceptable salt, a solvate and/or a hydrate thereof.

The present invention also relates to a method for treating a disease or an infection caused by an enterovirus in a mammal in need thereof, comprising administering to the mammal in need thereof a therapeutically effective amount of a pharmaceutical composition comprising the compound represented by Formula I, a geometric isomer thereof or a pharmaceutically acceptable salt, a solvate and/or a hydrate thereof, or the compound represented by Formula I, a geometric isomer thereof or a pharmaceutically acceptable salt, a solvate and/or a hydrate thereof.

The present invention also relates to a method for inhibiting the replication or reproduction of enterovirus in a mammal in need thereof, comprising administering to the mammal in need thereof an effective amount of a pharmaceutical composition comprising the compound represented by Formula I, a geometric isomer thereof or a pharmaceutically acceptable salt, a solvate and/or a hydrate thereof, or the compound represented by Formula I, a geometric isomer thereof or a pharmaceutically acceptable salt, a solvate and/or a hydrate thereof.

In some embodiments, in the pharmaceutical composition of the present invention, the compound represented by Formula I, a geometric isomer thereof or a pharmaceutically acceptable salt, a solvate and/or a hydrate thereof is present in an effective amount for treating a disease or an infection caused by an enterovirus.

In some embodiments, the pharmaceutical composition of the present invention further comprises a pharmaceutically acceptable carrier or excipient.

In some embodiments, the pharmaceutical composition of the present invention is a solid preparation, an injection, an external preparation, a spray, a liquid preparation or a compound preparation.

In some embodiments, the disease or infection caused by an enterovirus of the present invention includes, but is not limited to, hand-foot-and-mouth disease, viral angina, aseptic meningitis, brain stem encephalitis, myocarditis, neurogenic pulmonary edema and central nervous system infection.

In some embodiments, the cell of the present invention is a cell of mammal. In some embodiments, the mammal of the present invention includes bovine, equine, caprinae, suidae, canine, feline, rodent, primate, such as human, cat, dog or pig.

In some embodiments, the pharmaceutically acceptable salt of the compound represented by Formula I of the present invention includes inorganic or organic acid salt thereof, as well as inorganic or organic base salt thereof. The present invention relates to all forms of the above salts, including but not limited to, sodium salt, potassium salt, calcium salt, lithium salt, meglumine salt, hydrochloride, hydrobromide, hydroiodide, nitrate, sulfate, hydrosulfate, phosphate, hydrophosphate, acetate, propionate, butyrate, oxalate, pivalate, adipate, alginate, lactate, citrate, tartrate, succinate, maleate, fumarate, picrate, aspartate, gluconate, benzoate, methanesulfonate, ethanesulfonate, benzenesulfonate, p-toluenesulfonate and pamoate, etc.

The alkyl described in the present invention refers to a saturated straight-chain or branched monovalent hydrocarbonyl, preferably having 1-8 carbon atoms, such as 1-6, 1-4 or 1-3 carbon atoms. In some embodiments, the alkyl described in the present invention includes, but is not limited to, methyl, ethyl, n-propyl, isopropyl, n-butyl, isobutyl, tert-butyl, n-pentyl, tert-pentyl, neopentyl, hexyl, heptyl, octyl, etc.

In some embodiments, the alkyl described in the present invention is methyl, ethyl, n-propyl, isopropyl, n-butyl, isobutyl or tert-butyl.

In some embodiments, the alkyl described in the present invention is methyl, ethyl, n-propyl, isopropyl or n-butyl.

The ester group described in the present invention refers to R₁-C(O)-O-, wherein R₁ is selected from the alkyl described in the present invention. In some embodiments, the ester group described in the present invention includes but is not limited to CH₃-C(O)-O-, CH₃-CH₂-C(O)-O-, (CH₃)₂-CH-C(O)-O-, CH₃-(CH₂)₂-C(O)-O- or CH₃-(CH₂)₃-C(O)-O-, etc.

The alkoxy described in the present invention refers to group R₂-O-, wherein R₂ is the alkyl group described in the present invention. In some embodiments, the alkoxy described in the present invention includes but is not limited to methoxy, ethoxy, n-propoxy, isopropoxy, n-butoxy, tert-butoxy, sec-butoxy, n-pentoxy, n-hexyl, 1,2-dimethylbutoxy, etc. In some embodiments, the alkoxy described in the present invention includes but is not limited to methoxy, ethoxy, n-propoxy, isopropoxy or n-butoxy.

The heterocyclyl described in the present invention refers to a cycloalkyl with 3-8, 5-8, 3-6 or 5-6 carbon atoms containing one, two or more heteroatoms independently selected from N, O and S. Typical examples of "heterocyclyl" include but are not limited to tetrahydrofuryl, tetrahydrothienyl, pyrrolidinyl, piperazinyl, thiazinyl, piperidyl and morpholinyl, etc.

In some embodiments, R in Formula I described in the present invention is hydroxyl, hydrogen atom, R₁-C(O)-O-, C₁₋₈ alkoxy, phenyl, heterocyclyl or C₁₋₈ straight or branched alkyl, wherein R₁ is C₁₋₈ straight or branched alkyl.

In some embodiments, R in Formula I of the present invention is hydroxyl, hydrogen atom, R₁-C(O)-O-, C₁₋₆ alkoxy, phenyl, heterocyclyl, or C₁₋₆ straight or branched alkyl, wherein R₁ is C₁₋₆ straight or branched alkyl.

In some embodiments, R in Formula I of the present invention is hydroxyl, hydrogen atom, R₁-C(O)-O-, C₁₋₄ alkoxy, phenyl, heterocyclyl, or C₁₋₄ straight or branched alkyl, wherein R₁ is C₁₋₄ straight or branched alkyl.

In some embodiments, R in Formula I of the present invention is hydroxyl, hydrogen atom, methyl, ethyl, n-propyl, isopropyl, n-butyl, methoxy, ethoxy, n-propoxy, isopropoxy, n-butoxy, phenyl, CH₃-C(O)-O-, CH₃-CH₂-C(O)-O-, (CH₃)₂-CH-C(O)-O-, CH₃-(CH₂)₂-C(O)-O- or CH₃-(CH₂)₃-C(O)-O-.

In some embodiments, R in Formula I of the present invention is hydroxyl or (CH₃)₂-CH-C(O)-O- (i.e., isobutyryloxy group).

In some embodiments, R in Formula I of the present invention is R₁-C(O)-O-, wherein R₁ is C₁₋₈ straight or branched alkyl. In some embodiments, R in Formula I of the present invention is R₁-C(O)-O-, wherein R₁ is C₁₋₆ straight or branched alkyl. In some embodiments, R in Formula I of the present invention is R₁-C(O)-O-, wherein R₁ is C₁₋₄ straight or branched alkyl.

In some embodiments, R in Formula I of the present invention is C₁₋₈ alkoxy group. In some embodiments, R in Formula I of the present invention is C₁₋₆ alkoxy. In some embodiments, R in Formula I of the present invention is C₁₋₄ alkoxy.

In some embodiments, R in Formula I of the present invention is heterocyclyl.

In some embodiments, R in Formula I of the present invention is C₁₋₈ straight or branched alkyl. In some embodiments, R in Formula I of the present invention is C₁₋₆ straight or branched alkyl. In some embodiments, R in Formula I of the present invention is C₁₋₄ straight or branched alkyl.

In some embodiments, R in Formula I of the present invention is hydroxyl.

In some embodiments, R in Formula I of the present invention is hydrogen atom.

In some embodiments, R in Formula I of the present invention is methyl.

In some embodiments, R in Formula I of the present invention is ethyl.

In some embodiments, R in Formula I of the present invention is n-propyl.

In some embodiments, R in Formula I of the present invention is isopropyl.

In some embodiments, R in Formula I of the present invention is n-butyl.

In some embodiments, R in Formula I of the present invention is methoxy.

In some embodiments, R in Formula I of the present invention is ethoxy.

In some embodiments, R in Formula I of the present invention is n-propoxy.

In some embodiments, R in Formula I of the present invention is isopropoxy.

In some embodiments, R in Formula I of the present invention is n-butoxy.

In some embodiments, R in Formula I of the present invention is phenyl.

In some embodiments, R in Formula I of the present invention is CH₃-C(O)-O-.

In some embodiments, R in Formula I of the present invention is CH₃-CH₂-C(O)-O-.

In some embodiments, R in Formula I of the present invention is (CH₃)₂-CH-C(O)-O-.

In some embodiments, R in Formula I of the present invention is CH₃-(CH₂)₂-C(O)-O-.

In some embodiments, R in Formula I of the present invention is CH₃-(CH₂)₃-C(O)-O-.

In some embodiments, the compound represented by Formula I of the present invention is EIDD-2801.

In some embodiments, the compound represented by Formula I of the present invention is EIDD-1931.

The pharmaceutical composition of the present invention can be prepared into various forms according to different administration routes.

According to the present invention, the pharmaceutical composition can be administered in any one of the following routes: oral administration, spray inhalation, rectal administration, nasal administration, buccal administration, vaginal administration, topical administration, parenteral administration such as subcutaneous, intravenous, intramuscular, intraperitoneal, intrathecal, intraventricular, intrasternal and intracranial injection or infusion, or by means of an explanted reservoir. Among them, oral, intraperitoneal or intravenous administration is preferred.

When orally administered, the compound represented by Formula I, a geometric isomer, a pharmaceutically acceptable salt, a solvate and/or a hydrate thereof of the present invention can be prepared into any form of orally acceptable preparation, including but not limited to a tablet, a capsule, an aqueous solution or an aqueous suspension. The carrier for use in a tablet generally includes lactose and corn starch, and a lubricant such as magnesium stearate can also be added. The diluent for use in a capsule generally includes lactose and dry corn starch. The aqueous suspension is usually used by mixing an active ingredient with a suitable emulsifier and a suitable suspending agent. If necessary, a sweetener, a flavoring agent or a coloring agent can also be added to the above-mentioned forms of oral preparation.

When rectally administered, the compound represented by Formula I, a geometric isomer, a pharmaceutically acceptable salt, a solvate, and/or a hydrate thereof of the present invention can generally be prepared in a form of suppository, which is prepared by mixing the drug with a suitable non-irritating excipient. The excipient is present in solid state at room temperature, but melts at the rectal temperature to release the drug. Such excipient includes cocoa butter, beeswax and polyethylene glycol.

When topically administered, especially for treatment of easily accessible affected-surface or organ, such as eye, skin, or lower intestinal neurological disease by topical application, the compound represented by Formula I, a geometric isomer, a pharmaceutically acceptable salt, a solvate and/or a hydrate thereof of the present invention can be prepared in various forms of topical preparations according to different affected-surfaces or organs, the specific instructions are as follows:
When topically administered to eye, the compound represented by Formula I, a geometric isomer, a pharmaceutically acceptable salt, a solvate and/or a hydrate thereof of the present invention can be formulated into a preparation form such as micronized suspension or solution, the carrier used is isotonic sterile saline with a certain pH, and a preservative such as benzyl chloride alkoxide may or may not be added. In addition, for administration to eye, the compound can also be prepared in a form of ointment such as vaseline ointment.

When topically administered to skin, the compound represented by Formula I, a geometric isomer, a pharmaceutically acceptable salts, a solvate and/or a hydrate thereof of the present invention can be prepared into a suitable form such as an ointment, a lotion or a cream, in which the active ingredient is suspended or dissolved in one or more carriers. The carrier for use in an ointment includes, but is not limited to: mineral oil, liquid petrolatum, white petrolatum, propylene glycol, polyethylene oxide, polypropylene oxide, emulsifying wax, and water. The carrier for use in a lotion or a cream includes, but is not limited to: mineral oil, sorbitan monostearate, Tween-60, cetyl ester wax, hexadecenyl aryl alcohol, 2-octyldodecanol, benzyl alcohol and water.

When topically administered to lower intestinal tract, the compound represented by Formula I, a geometric isomer, a pharmaceutically acceptable salt, a solvate and/or a hydrate thereof of the present invention can be prepared into a form such as rectal suppository as described above or a suitable enema preparation form, in addition, a topical transdermal patch can also be used.

The compound represented by Formula I, a geometric isomer, a pharmaceutically acceptable salt, a solvate and/or a hydrate thereof of the present invention can also be administered in a preparation form of sterile injection, including sterile injectable aqueous solution or oil suspension, or sterile injectable solutions, wherein the usable carrier and solvent includes water, Ringer's solution and isotonic sodium chloride solution. In addition, a sterilized non-volatile oil such as monoglyceride or diglyceride can also be used as solvent or suspension media.

The drugs of the above various preparation forms can be prepared according to conventional methods in the pharmaceutical field.

As used in the present invention, the term "effective amount" refers to an amount sufficient to achieve the desired therapeutic or preventive effect, for example, an amount to achieve the alleviation of symptoms associated with the disease to be treated (such as a disease or an infection caused by an enterovirus), or an amount that can effectively prevent, suppress or delay the occurrence of a disease (such as a disease or an infection caused by an enterovirus). Determination of such an effective amount is within the capabilities of those skilled in the art.

As used in the present invention, the term "treatment" aims to alleviate, reduce, improve or eliminate the disease state or condition targeted. If a subject receives a therapeutic amount of the compound represented by Formula I, a geometric isomer, a pharmaceutically acceptable salt, a solvate and/or a hydrate thereof or the pharmaceutical composition according to the method described herein, and one or more signs and symptoms of the subject show an observable and/or detectable reduction or improvement, the subject is successfully "treated". It should also be understood that the treatment of disease state or condition includes not only complete treatment, but also incomplete treatment in which some biological or medical results are achieved.

In addition, it should be noted that the specific dosage and method of using the compound represented by Formula I, a geometric isomer, a pharmaceutically acceptable salt, a solvate and/or a hydrate thereof described in the present invention depend on many factors, including the patient's age, weight, gender, natural health status, nutritional status, active strength of compound, administration time, metabolic rate, severity of disease, and subjective judgment of physician. Herein it is preferred to use a dosage between 0.01-1000 mg/kg body weight/day.

The compound represented by Formula I, a geometric isomer, a pharmaceutically acceptable salt, a solvate and/or a hydrate thereof described in the present invention can inhibit enterovirus replication in cells and reduce the viral nucleic acid load in cell culture.

The compound represented by Formula I, a geometric isomer, a pharmaceutically acceptable salt, a solvate and/or a hydrate thereof described in the present invention has one or more of the following biological activities:
1) anti-enterovirus activity,
2) enhancing or improving the viability or survival rate of enterovirus-infected cells.

The above-mentioned biological activity of the compound represented by Formula I, a geometric isomer, a pharmaceutically acceptable salt, a solvate and/or a hydrate thereof described in the present invention is achieved via at least one of the following pathways:
A. inhibiting the CPE level of enterovirus-infected cells;
B. inhibiting the replication of enterovirus RNA and the formation of infectious virus particles;
C. inhibiting the production of enterovirus proteins;
D. playing an antiviral role in the viral replication stage.

### Brief Description of the Drawings

Figure 1 shows the structural formulas of compounds EIDD-1931 and EIDD-2801.
Figure 2 shows the protection effect of compounds EIDD-1931 and EIDD-2801 on CPE caused by EV-A71 in different cell lines, showing that EIDD-1931 and EIDD-2801 could dose-dependently inhibit the cytopathic effect caused by the virus in multiple cell lines infected with EV-A71.
Figure 3 shows the experimental results of the inhibition of compounds EIDD-1931 and EIDD-2801 on EV-A71 RNA replication and infectious virus particle formation, showing that EIDD-1931 and EIDD-2801 both dose-dependently inhibited the replication of EV-A71 RNA (B and D), and also inhibited the production of infectious virus particles (A and C).
Figure 4 shows the experimental results of the inhibition of compounds EIDD-1931 and EIDD-2801 on EV-A71 VP1 protein synthesis, showing that both EIDD-1931 and EIDD-2801 could inhibit the production of EV-A71 VP1 protein.
Figure 5 shows the experimental results of the mechanism of action of compound EIDD-1931 against EV-A71, showing that EIDD-1931 exerts an antiviral effect during the replication stage of EV-A71.
Figure 6 shows the protective effects of compounds EIDD-1931 and EIDD-2801 on 1-day-old ICR suckling mice with lethal infection, showing that both EIDD-1931 and EIDD-2801 could improve the survival rate of 1-day-old ICR suckling mice infected with EV-A71.
Figure 7 shows that both compounds EIDD-1931 and EIDD-2801 could reduce the viral load of tissues and organs of 1-day-old ICR suckling mice infected with EV-A71.

### Specific Models for Carrying Out the Invention

The substantive content of the present invention is further described in combination with the specific examples of the present invention. It should be understood that the following examples are only used to illustrate the present invention, but are not used to limit the scope of protection of the present invention. If the specific conditions are not specified in the following examples, they were carried out according to conventional conditions or the manufacturer's recommendations. The drugs or reagents used without indicating the manufacturer were all conventional products that could be purchased commercially.

Although many of the materials and operating methods used in the following examples were well known in the art, they were still described as detailed as possible in the present invention. It was clear to those skilled in the art that, unless otherwise specified, the materials and operating methods used in the following examples were well known in the art.

### Example 1. Evaluation of in vitro anti-EV-A71 activity of compounds EIDD-1931 and EIDD-2801 based on cytopathic effect

### (1) Experimental materials

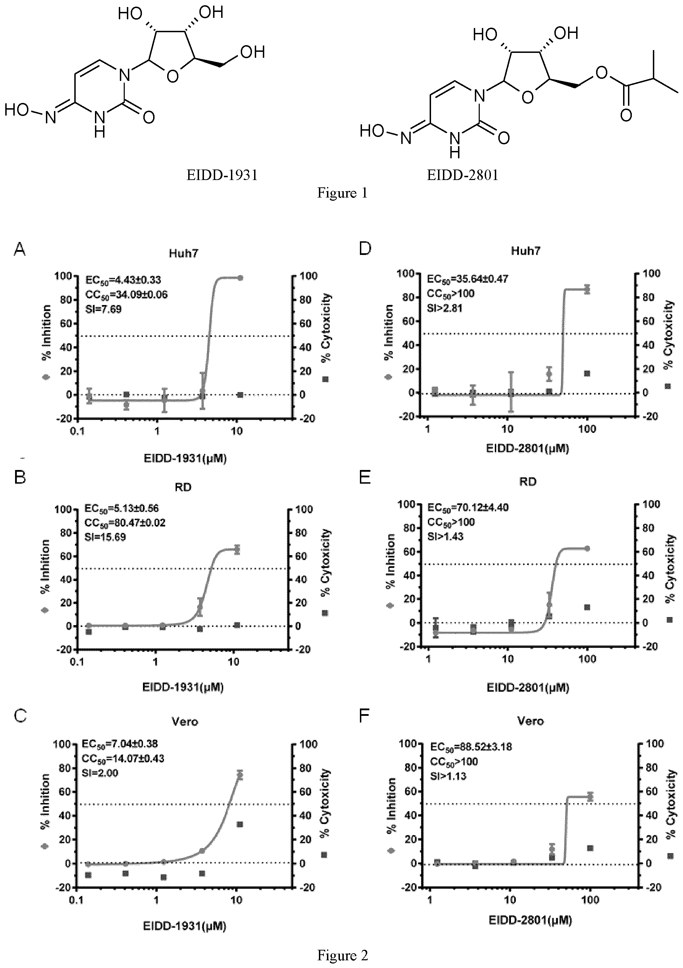

Human liver tumor cells (Huh7 cells) used in the experiment were purchased from Chinese National Infrastructure of Cell Line Resource. Human rhabdoid tumor cells (RD cells) and African green monkey kidney cells (Vero cells) were purchased from ATCC (Cat. No.: CCL-136 and CCL-81, respectively). The above cells were cultured in a cell culture incubator at 37°C and 5% CO₂. The complete culture medium used for cell growth was DMEM high-glucose culture medium (purchased from Gibco, Cat. No.: 2367374) supplemented with 10% FBS (purchased from Gibco, Cat. No.: 16000044) and penicillin-streptomycin (purchased from Gibco, Cat. No.: 2321152). The maintenance culture medium used for cell culture was DMEM high-glucose culture medium supplemented with 2% FBS and penicillin-streptomycin. H strain of EV-A71 was purchased from ATCC (Cat. No.: VR-1432). Compounds EIDD-1931 and EIDD-2801 were purchased from Shanghai Topscience Biochemical Technology Co., Ltd. (Cat Nos: T8498 and T8309, respectively), and their structures were shown in Figure 1. Cell-Titer Glo^{®} Luminescent Cell Viability Assay Solution was purchased from Promega (Cat. No.: G7572). The microplate reader, model Spectra Max M5, was purchased from Molecular Devices.

### (2) The experimental scheme was as follows:

Huh7 cells, RD cells, and Vero cells were seeded into a 96-well plate with a white wall and a transparent bottom at a density of about 1.0×10⁴ per well and cultured at 37°C and 5% CO₂ for 24 hours. Then, the EV-A71 virus was diluted with cell maintenance solution and added to the 96-well plate to reach a final concentration of 100TCID₅₀. At the same time, the test compounds EIDD-2801 and EIDD-1931 were diluted with cell maintenance solution and added to the 96-well plate to reach a final concentration of 100µM, 33µM, 11µM, 0.37µM, 0.12µM, 0.04µM, 0.01µM and 0.005µM, respectively. After 3 days, the supernatant was discarded, and the diluted Cell-Titer Glo^{®} Luminescent Cell Viability Assay Solution was added. The cells were shaken and lysed for 5 minutes in the dark, and then allowed to stand for 3 minutes. Finally, the luminescence value of each well was measured using the microplate reader.

The formula for calculating the inhibition rate of the test compounds against virus was: Inhibition rate (%) = (value of experimental group - average value of virus group) / (average value of cell control group - average value of virus group) × 100

### (3) Cytotoxicity test of compounds EIDD-1931 and EIDD-2801 on three different cell lines

The experimental scheme was as follows:
Huh7 cells, RD cells, and Vero cells were seeded in a 96-well plate with a white wall and transparent bottom at a density of 1.0× 10⁴ per well and cultured at 37°C and 5% CO₂ for 24 hours. The test compounds EIDD-1931 and EIDD-2801 were diluted in multiples with cell maintenance solution and then added to the 96-well plate so that the final concentrations thereof were 100µM, 33µM, 11µM, 0.37µM, 0.12µM, 0.04µM, 0.01µM and 0.005µM, respectively. After 3 days, the supernatant was discarded, and the diluted Cell-Titer Glo^{®} Luminescent Cell Viability Assay Solution was added. The cells were shaken and lysed for 5 minutes in the dark, and then allowed to stand for 3 minutes. Finally, the luminescence value of each well was measured using the microplate reader.

The inhibition rate of the test compounds at each dilution was calculated as follows: Inhibition rate (%) = (average value of cell control group - value of experimental group) / average value of cell control group × 100

### (4) Data analysis

Origin9.0 software was used to fit the inhibition rate-concentration S-shaped curve and calculate the half-maximal effective concentrations (EC₅₀) of the test compounds. The half-cytotoxic concentrations (CC₅₀) of the test compounds were calculated using the same method, and the select index (SI) was calculated based on EC₅₀ and CC₅₀: SI = CC₅₀/EC₅₀.

### (5) Experimental results

The experimental results were shown in Figure 2. EIDD-1931 and EIDD-2801 could dose-dependently inhibit the cytopathic effect caused by the virus in multiple cell lines infected with EV-A71. The EC₅₀ value, CC₅₀ value, and SI of EIDD-1931 on RD cells were 5.13±0.56 µM, 80.47±0.02 µM, and 15.69, respectively; the EC₅₀ value, CC₅₀ value, and SI of EIDD-1931 on Vero cells were 7.04±0.38µM, 14.07±0.43 µM, and 2.0, respectively; and the EC₅₀ value, CC₅₀ value, and SI of EIDD-1931 on Huh7 cells were 4.43±0.33µM, 34.09±0.06 µM, and 7.69, respectively. The EC₅₀ value, CC₅₀ value, and SI of EIDD-2801 on RD cells were 70.12±4.40 µM, >100µM, and >1.43, respectively; the EC₅₀ value, CC₅₀ value, and SI of EIDD-2801 on Vero cells were 88.52±3.18 µM, >100µM, and >1.13, respectively; and the EC₅₀ value, CC₅₀ value, and SI of EIDD-2801 on Huh7 cells were 35.64±0.47 µM, >100µM, and >2.81, respectively.

### Example 2. Experiment on inhibition of compounds EIDD-1931 and EIDD-2801 on EV-A71 viral RNA replication and infectious virus particle formation in supernatant

### (1) Experimental materials

Some experimental materials were the same as above, and the additional experimental materials were as follows: RNeasy^{®} Mini kit was purchased from Qiagen (Cat. No. 74106), One Step Prime Script^{™} RT-PCR kit was purchased from Takara (Cat. No. RR47Q), and the standard plasmid of H strain of EV-A71 H and RT-PCR primers were synthesized by Sangon Biotech (Shanghai) Co., Ltd.

### (2) Experimental method:

1. RD cells were seeded in a 12-well plate at a density of about 4.0×10⁵ per well and cultured at 37°C and 5% CO₂ for 24 hours. Subsequently, the EV-A71 virus was diluted to 0.1 MOI with cell maintenance medium and added to the 12-well plate. At the same time, the test compounds EIDD-1931 and EIDD-2801 were diluted to corresponding concentrations with cell maintenance medium and added to the 12-well plate, so that the final concentrations of EIDD-1931 were 20 µM, 10 µM, 5 µM, 2.5 µM and 1.25 µM, respectively, and the final concentrations of EIDD-2801 were 200 µM, 100 µM, 50 µM, 25 µM and 12.5 µM, respectively. The cells were cultured at 37°C, 5% CO₂ for 1.5 hours, then the supernatant was discarded, 1 mL of the diluted test compound was added to each well, and continuously cultured for 30 hours, the supernatant was collected when the cells begin to show cytopathy, centrifuged at 8000 rpm for 5 min, aliquoted, and stored at -80°C for later use.

### 2. RNA extraction

After 30 hours, total intracellular RNA was extracted according to the instructions of the RNeasy^{®} Mini kit.

### 3. qRT-PCR detection

1) Standards were prepared with recombinant EV-A71 linearized plasmid: the copy numbers of the standards were calculated according to the concentrations and molecular weight of the linearized plasmid, and dilution was accurately performed to -1, -2, -3, -4, -5, -6, -7, -8, -9..., until the copy number was lower than the detection limit of the fluorescence quantitative PCR instrument. After the standards were prepared, the standards of the concentrations were aliquoted, and cryopreserved at -80°C for later use.
2) One Step Prime Script^{TM} RT-PCR kit was used to detect the RNA load of EV-A71 virus in the sample. The primer and probe sequences used in the experiment were as follows:
   EV71-RT-F: 5'-CCAATCTCAGCGGCTTGGAG-3'
   EV71-RT-R: 5'-CACTCAAGCTCTACCGGCAC-3'
   EV71-RT-Probe: FAM-TCCAATCGATGGCTGCTCACCTGCGT-BHQ1.
3) The reaction procedure was as follows:
   reverse transcription: 42°C 5 minutes;
   pre-denaturation: 95°C 3 minutes;
   signal acquisition: 95°C 20 seconds, 59°C 1 minute, a total of 45 cycles. Inhibition rate (%) = (RNA copy number of drug-treated group) / (RNA copy number of cell infection group) × 100

### 4. TCID₅₀ detection

RD cells were seeded in a 96-well plate at a density of about 1.0×10⁴ per well and cultured at 37°C and 5% CO₂ for 24 hours. The cell culture medium was then discarded and 150µL of cell maintenance medium was added to each well. The supernatant of each group obtained in step 1 was diluted with cell maintenance medium (starting from 10⁻¹, 10-fold dilution, a total of 8 gradients), and then the diluted supernatant was added to the 96-well plate, 50µL/well, 4 replicates for each dilution, and 50 µL of cell maintenance medium was added to the cell control group. Then the cells were cultured for 5 days, and the TCID₅₀ value of each sample was calculated using the Reed-Muench method.

### (3) Statistical analysis

Statistical significance was calculated using analysis of variance (ANOVA). Data were presented as mean ± standard deviation. p<0.05 indicated statistical significance.

### (4) Experimental results

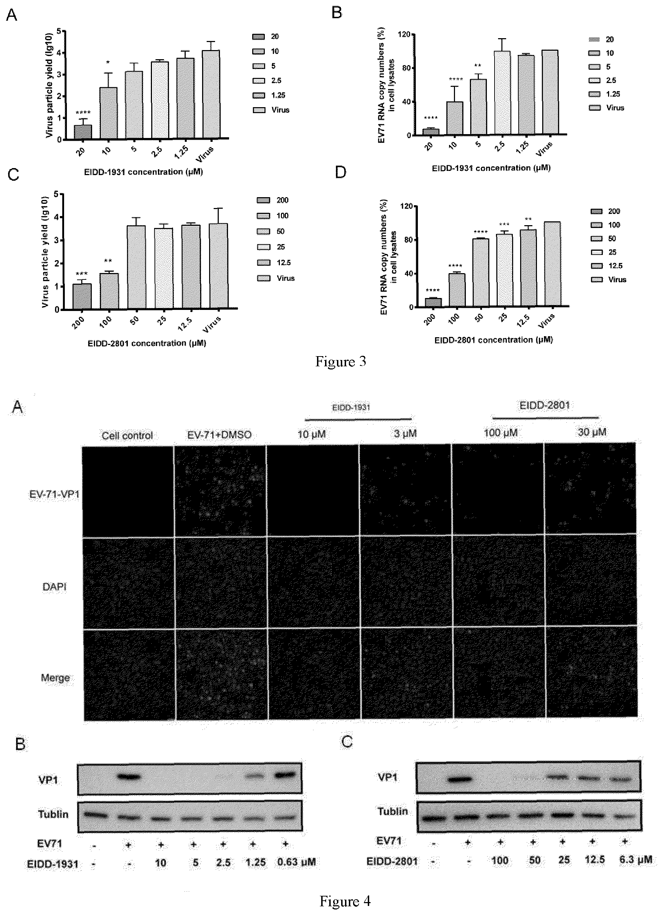

As shown in Figure 3, compounds EIDD-1931 and EIDD-2801 both dose-dependently inhibited the replication of EV-A71 RNA (B and D), and also inhibited the production of infectious viral particles (A and C).

### Example 3. Experiment on inhibition of compounds EIDD-1931 and EIDD-2801 on EV-A71 VP1 protein synthesis

### (1) Experimental materials

Some experimental materials were the same as above, and the additional experimental materials were as follows: mouse anti-EV-A71 VP1 antibody (primary antibody) was donated by Professor Cheng Tong of Xiamen University, mouse anti-alpha tubulin antibody and HRP-conjugated goat anti-mouse IgG antibody were purchased from Abcam (Cat Nos: ab7291 and ab205719, respectively), Alexa Fluor 647 donkey anti-mouse IgG (H+L) secondary antibody and cell nucleus dye Hoechst 33342 were purchased from Invitrogen (Cat Nos: A31571 and H21492, respectively), and RIPA lysis buffer, skim milk powder, BCA protein quantification kit, Super Ecl luminescent liquid with ultra-high sensitivity were purchased from APPLYGEN (Cat Nos: P1622, P1511, C1053 and P1050).

### (2) Immunofluorescence (IF) experiment

Vero cells were seeded in a 96-well black plate at a density of about 1.0×10⁴ per well and cultured at 37°C and 5% CO₂ for 24 hours. Then, EV-A71 virus was diluted to the corresponding concentration with cell maintenance medium and added into the 96-well plate (MOI=1). At the same time, the test compounds EIDD-1931 and EIDD-2801, which had been diluted in advance with cell maintenance medium, were added at the final concentrations of 10 µM, 3 µM and 100 µM, 30 µM, respectively. Then the cells were cultured for 16 hours, the liquid was discarded, 100 µL of 4% formaldehyde was added to each well for fixation for 30 minutes, the fixative was discarded, PBS buffer was added, and the antibody was incubated and labeled for imaging.

### (3) Western blot experiment

RD cells were seeded in a 12-well plate at a density of about 1.0×10⁵ per well and cultured at 37°C and 5% CO₂ for 24 hours. Then, EV-A71 virus was diluted to the corresponding concentration with cell maintenance medium and added to the 12-well plate so that the virus content in each well was 0.1 MOI. At the same time, EIDD-1931 and EIDD-2801 were diluted to the corresponding concentration with cell maintenance medium and added to the 12-well plate so that the final concentrations of EIDD-1931 were 10 µM, 5 µM, 2.5 µM, 1.25 µM, and 0.63 µM, respectively, and the final concentrations of EIDD-2801 were 100 µM, 50 µM, 25 µM, 12.5 µM, and 6.3 µM, respectively. Then the cells were cultured for 24 hours, and then the supernatant was discarded, and the intracellular proteins were collected for analysis by Western blot. The specific steps were as follows: adherent cells were fully lysed with cell lysis buffer (RIPA) (100 µL/well) to obtain whole cell protein. After the protein concentration was determined with BCA protein quantification kit, the corresponding volume of 5 × SDS Loading Buffer was added and boiled at 100°C for 10 minutes to fully denature the protein. Then, 10% SDS-PAGE was used for electrophoresis at 80V/120V for 2 hours to separate proteins of different sizes. The protein was transferred to PVDF membrane at 300mA for 90 minutes to fully be transferred to the membrane. After the transfer, the membrane was cut into appropriate sizes according to the molecular weight of the target protein, and blocked with 5% skim milk powder diluted in TBST at room temperature for 1 hour. The primary antibodies, anti-EV-A71 VP1 protein antibody (1:500 dilution) and internal reference Tublin antibody (1:5000 dilution), diluted in the blocking solution were added, and incubated at 4°C overnight by a shaker. Then, HRP-conjugated goat anti-mouse IgG antibody (1:5000 dilution) was added and incubated at room temperature for 1 hour. Finally, the ultrasensitive chemiluminescence method was used for color development and photography.

### (4) Experimental results

The experimental results were shown in Figure 4. Both EIDD-1931 and EIDD-2801 could dose-dependently inhibit the production of EV-A71 VP1 protein. As the concentration of the tested compound decreases, the inhibitory effect gradually weakened.

### Example 4. Experimental verification of action mechanism of compound EIDD-1931 against EV-A71 virus

### (1) Experimental materials

The experimental materials used herein were the same as above.

### (2) Time series experiment

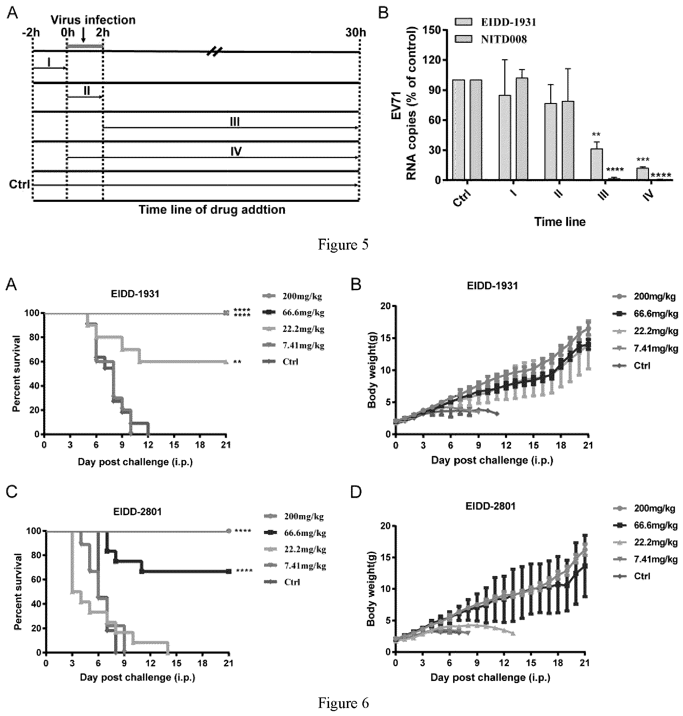

The concentration of EIDD-1931 used in the experiment was 10µM, the concentration of the positive compound NITD008 was 2µM, and the infection dose of EV-A71 was 0.01 MOI. The specific steps were as follows: RD cells were seeded in a 12-well plate at a density of about 4.0×10⁵ per well and cultured at 37°C and 5% CO₂ for 24 hours. Subsequently, the test compound EIDD-1931, the positive compound NITD008 and the EV-A71 virus were diluted to the corresponding concentrations with the cell maintenance medium, and then the pre-diluted compound or virus was added at the time point as shown in A in Figure 5. After 30 hours of continuous culture, the cell supernatant was discarded, the intracellular RNA was extracted, and the intracellular viral RNA load was detected by qRT-PCR. The intracellular RNA extraction method and qRT-PCR system and reaction conditions were the same as described above.

### (2) Statistical analysis

Statistical significance was calculated using analysis of variance (ANOVA). The data were presented in the form of mean ± standard deviation. p < 0.05 indicated statistical significance.

### (3) Experimental results

The experimental results were shown in Figure 5. The compound EIDD-1931 mainly exerted an antiviral effect during the replication stage of EV-A71 virus. After adding EIDD-1931 to RD cells at different stages of EV-A71 virus infection, the EV-A71 viral RNA load in the cells was detected. It showed that EIDD-1931 mainly played a role in the viral replication stage and had no obvious inhibition on the adsorption and entry of the virus.

### Example 5. Experiment on protection of compounds EIDD-1931 and EIDD-2801 to 1-day-old ICR suckling mice with lethal infection

### (1) Experimental materials

The experimental materials used herein were the same as above.

### (2) Mouse strains

The 1-day-old ICR suckling mice used in the experiment were SPF grade and purchased from Beijing Vital River Laboratory Animal Technology Co., Ltd, the source of which was clear, and which was passed the inspection.

### (3) Experimental procedures

10 litters of 1-day-old ICR offspring female mice were randomly divided into 10 groups, namely, virus control group (2 groups), EIDD-1931 administration group (4 groups), and EIDD-2801 administration group (4 groups). The dosages of EIDD-1931 and EIDD-2801 were 200 mg/kg, 66.6 mg/kg, 22.2 mg/kg, and 7.41 mg/kg, respectively. Each mouse was challenged with 10⁶ PFU intraperitoneally, and administrated the drug intraperitoneally 4 hours later. The mice of virus control group were administrated the drug solvent. And then, the drug was administered once per day for 7 consecutive days. The mice were weighed, and the survival and weight changes in the mice were recorded every day.

### (4) Statistical analysis

Log-Rank was used to calculate the statistical significance of the survival curve. p<0.05 indicated statistical significance.

### (5) Experimental results

The experimental results were shown in Figure 6. Compounds EIDD-1931 and EIDD-2801 showed a dose-dependent protective effect on ICR suckling mice inoculated with a lethal dose of EV-A71 virus. EIDD-1931 at doses of 200 mg/kg and 66.6 mg/kg could completely protect 1-day-old ICR suckling mice from the lethal dose of EV-71 virus, and EIDD-1931 at dose of 22.2 mg/kg could protect 1-day-old ICR suckling mice from the lethal dose of EV-71 virus with survival rate of 60%. The mice that survived at these three dosing concentrations were able to maintain good weight gain, while the mice in the control group or low-dose group (7.41 mg/kg) died within 12 days (A and B in Figure 6). EIDD-2801 at 200 mg/kg and 66.6 mg/kg could protect 1-day-old ICR suckling mice with survival rate of 100% and 67%, respectively, while the mice in the 22.2 mg/kg and 7.41 mg/kg dose groups, like the control group, all died within 13 days (C and D in Figure 6).

### Example 6. Experiment on inhibition of compounds EIDD-1931 and EIDD-2801 on viral load in suckling mice infected with EV-A71

### (1) Experimental materials

Some experimental materials were the same as above, and the additional experimental materials required were as follows: TRIzol reagent was purchased from Invitrogen, Cat. No.: 15596026.

### (2) Mouse strain

The 1-day-old ICR suckling mice used in the experiment were SPF grade, purchased from Beijing Vital River Laboratory Animal Technology Co., Ltd, the source of which was clear, and which was passed the inspection.

### (3) Detection of EV-A71 viral load in ICR suckling mice tissues

Three litters of 1-day-old ICR offspring female mice were randomly divided into 3 groups, namely, the virus control group, the EIDD-1931 administration group (200 mg/kg), and the EIDD-2801 administration group (200 mg/kg). Each mouse was challenged with 10⁶ PFU intraperitoneally, and administrated the drug intraperitoneally 4 hours later. The mice of virus control group were administrated the drug solvent. And then, the drug was administered once per day for 4 consecutive days. The mice were euthanized on the 4^{th} day, and the brain, heart, small intestine, liver, hind limb muscle and lung tissues of the mice were collected. RNA in tissues was extracted using TRIzol reagent, and then the viral RNA in each tissue was quantitatively analyzed by qRT-PCR.

### (4) Statistical analysis

Statistical significance analysis was performed using an unpaired *t*-test. p<0.05 indicated statistical significance.

### (5) Experimental results

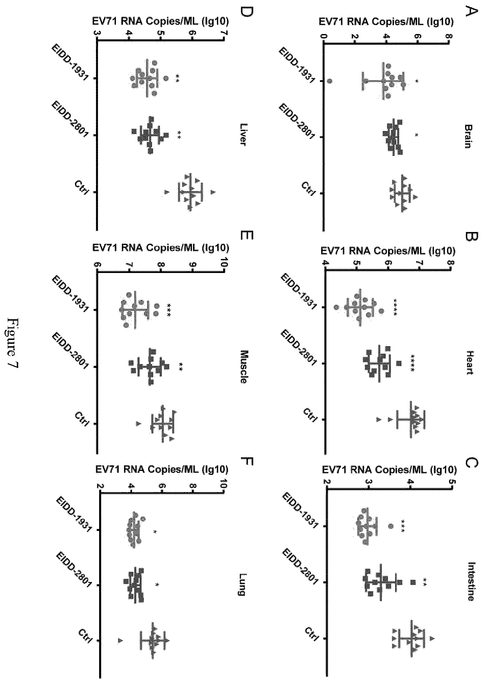

As shown in Figure 7, after the treatment with compounds EIDD-1931 and EIDD-2801, the viral loads in the tissues or organs of mice, such as brain, heart, small intestine, liver, hind limb muscle and lung, were significantly reduced. The above results showed that EIDD-1931 and EIDD-2801 had anti-EV-A71 virus activity *in vivo.*

### Example 7. Experiment on evaluating broad-spectrum anti-enteroviral activity of compounds EIDD-1931 and EIDD-2801

### (1) Experimental materials

Some experimental materials were the same as above, and the additional materials were as follows: EV-A71 AH08/06 strain and CV-B3 Nancy strain were all stored in this laboratory, and EV-D68 STL-2014-12 strain, CV-A6 TW-2007-00141 strain and CV-A16 190-D1 strain were all donated by Professor Cheng Tong of Xiamen University.

### (2) Experimental study on protection of EIDD-1931 and EIDD-2801 against cytopathic effect (CPE) caused by various enteroviruses

In this experiment, the experiment on protection of EIDD-1931 and EIDD-2801 against CPE caused by EV-A71 virus AH08/06 strain, EV-D68 virus STL-2014-12 strain, CV-A6 TW-2007-00141 strain, and CV-A16 190-D1 strain were all performed by using RD cells. The experiments on protection of EIDD-1931 and EIDD-2801 against CPE caused by CV-B3 Nancy strain were performed by using Vero cells. The specific experimental methods were the same as those in Example 1.

### (3) Experimental results

The experimental results showed that EIDD-1931 could inhibit the infection of various enteroviruses, and its EC₅₀ values were all less than 20µM, while EIDD-2801 had low inhibitory activity against EV-V68, CV-A16 and CV-B3, and its EC₅₀ value against CV-A6 was greater than 100µM.

**Table 1. Broad-spectrum anti-enteroviral activity of EIDD-1931 and EIDD-2801**

| Enterovirus virus | EIDD-1931 | | EIDD-2801 | |
|---|---|---|---|---|
| | EC₅₀(µM) | SI | EC₅₀(µM) | SI |
| EV-A71 AH08/06 | 2.79±0.89 | 12.22 | 30.29±0.55 | >3.3 |
| EV-D68 STL-2014-12 | 9.69±0.04 | 8.3 | 86.21±9.17 | >1.6 |
| CV-A6 TW-2007-00141 | 17.72±0.38 | 5.12 | >100 | - |
| CV-A16 190-D1 | 4.14±0.27 | 19.43 | 44.91±4.23 | >2.23 |
| CV-B3 Nancy | 3.65±0.82 | 3.85 | 87.23±10.84 | >1.15 |

Although the present invention has been described in detail above with general descriptions and specific embodiments, it is obvious to those skilled in the art that some modifications or improvements can be made to the present invention. Therefore, these modifications or improvements made without departing from the spirit of the present invention are within the scope of protection of the present invention.

## Claims

1. Use of a compound represented by Formula I, a geometric isomer thereof or a pharmaceutically acceptable salt, a solvate and/or a hydrate thereof in the manufacture of a medicament for treating a disease or an infection caused by an enterovirus (including but not limited to hand-foot-and-mouth disease, viral angina, aseptic meningitis, brain stem encephalitis, myocarditis, neurogenic pulmonary edema and central nervous system infection), 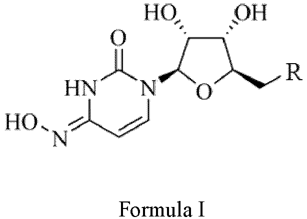 wherein R is hydroxyl, hydrogen atom, ester group, alkoxy, phenyl, heterocyclyl or alkyl.

2. Use of a pharmaceutical composition in the manufacture of a medicament for treating a disease or an infection caused by an enterovirus (e.g., hand-foot-and-mouth disease, viral angina, aseptic meningitis, brain stem encephalitis, myocarditis, neurogenic pulmonary edema and central nervous system infection), wherein the pharmaceutical composition comprises a compound represented by Formula I, a geometric isomer thereof or a pharmaceutically acceptable salt, a solvate and/or a hydrate thereof, 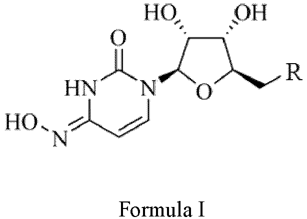
wherein R is hydroxyl, hydrogen atom, ester group, alkoxy, phenyl, heterocyclyl or alkyl,
preferably, the pharmaceutical composition further comprises a pharmaceutically acceptable carrier or excipient, specifically, the pharmaceutical composition is a solid preparation, an injection, an external preparation, a spray, a liquid preparation, or a compound preparation.

3. Use of a pharmaceutical composition in the manufacture of a medicament as an enterovirus inhibitor, wherein the pharmaceutical composition comprises a compound represented by Formula I, a geometric isomer thereof or a pharmaceutically acceptable salt, a solvate and/or a hydrate thereof, 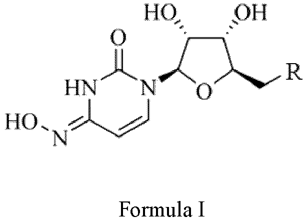 wherein R is hydroxyl, hydrogen atom, ester group, alkoxy, phenyl, heterocyclyl or alkyl, preferably, the pharmaceutical composition further comprises a pharmaceutically acceptable carrier or excipient, specifically, the pharmaceutical composition is a solid preparation, an injection, an external preparation, a spray, a liquid preparation, or a compound preparation.

4. Use of a pharmaceutical composition in the manufacture of a medicament for inhibiting the replication or reproduction of enterovirus in a cell (e.g., a cell of mammal), wherein the pharmaceutical composition comprises a compound represented by Formula I, a geometric isomer thereof or a pharmaceutically acceptable salt, a solvate and/or a hydrate thereof, 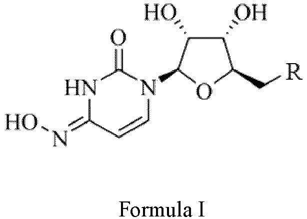
wherein R is hydroxyl, hydrogen atom, ester group, alkoxy, phenyl, heterocyclyl or alkyl,
preferably, the pharmaceutical composition further comprises a pharmaceutically acceptable carrier or excipient, specifically, the pharmaceutical composition is a solid preparation, an injection, an external preparation, a spray, a liquid preparation, or a compound preparation.

5. Use of a compound represented by Formula I, a geometric isomer thereof or a pharmaceutically acceptable salt, a solvate and/or a hydrate thereof in the manufacture of a medicament as an enterovirus inhibitor, 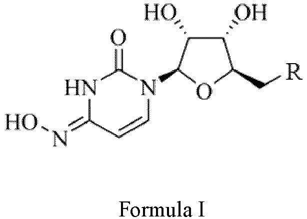 wherein R is hydroxyl, hydrogen atom, ester group, alkoxy, phenyl, heterocyclyl or alkyl.

6. Use of a compound represented by Formula I, a geometric isomer thereof or a pharmaceutically acceptable salt, a solvate and/or a hydrate thereof in the manufacture of a medicament for inhibiting the replication or reproduction of enterovirus in a cell (e.g., a cell of mammal), 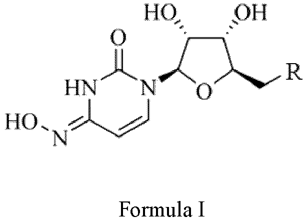 wherein R is hydroxyl, hydrogen atom, ester group, alkoxy, phenyl, heterocyclyl or alkyl.

7. The use according claim 4 or 6, wherein it is achieved by the medicament via at least one of the following pathways:
A. inhibiting the CPE level of enterovirus-infected cells;
B. inhibiting the replication of enterovirus RNA and the formation of infectious virus particles;
C. inhibiting the production of enterovirus VP1 protein;
D. playing an antiviral role after the viral adsorption stage.

8. The use according to any one of claims 1 to 6, wherein R is hydroxyl, hydrogen atom, R₁-C(O)-O-, C₁₋₈ alkoxy, phenyl, heterocyclyl, or C₁₋₈ straight or branched alkyl, wherein R₁ is C₁₋₈ straight or branched alkyl;
preferably, R is hydroxyl, hydrogen atom, R₁-C(O)-O-, C₁₋₆ alkoxy, phenyl, heterocyclyl, or C₁₋₆ straight or branched alkyl, wherein R₁ is a C₁₋₆ straight or branched alkyl;
preferably, R is hydroxyl, hydrogen atom, R₁-C(O)-O-, C₁₋₄ alkoxy, phenyl, heterocyclyl, or C₁₋₄ straight or branched alkyl, wherein R₁ is C₁₋₄ straight or branched alkyl;
preferably, R is hydroxyl, hydrogen atom, methyl, ethyl, n-propyl, isopropyl, n-butyl, methoxy, ethoxy, n-propoxy, isopropoxy, n-butoxy, phenyl, CH₃-C(O)-O-, CH₃-CH₂-C(O)-O-, (CH₃)₂-CH-C(O)-O-, CH₃-(CH₂)₂-C(O)-O-, or CH₃-(CH₂)₃-C(O)-O-;
preferably, R is hydroxyl or (CH₃)₂-CH-C(O)-O-.
